# EUROPEAN PATENT APPLICATION

(11) **EP 4 834 277 A1**
(43) Date of publication of application: **04.11.2026**
(21) Application number: 24910383.9
(22) Date of filing: 15.11.2024
(51) Int. Cl.: A61F 2/07

(54) **ENDOLUMINAL STENT AND STENT SYSTEM**

(30) Priority: 27.12.2023 CN 202311838045
(71) Applicant: Lifetech Scientific (Shenzhen) Co., Ltd., Shenzhen, Guangdong 518063 (CN)
(72) Inventor: LIN, Zhipeng, Shenzhen, Guangdong 518063 (CN); WANG, Wan, Shenzhen, Guangdong 518063 (CN)
(74) Representative: Michalski Hüttermann & Partner mbB
(86) International application number: PCT/CN2024/132267
(87) International publication number: WO 2025/139448

(57) **Abstract**

A lumen stent and a stent system are provided. The lumen stent (100) includes a stent main body (10), a semi-deployed structure (30), and an imaging assembly (40). The semi-deployed structure (30) includes a restraining unit (31) and a restraining point (33). The lumen stent (100) includes a first region (100a) and a second region (100b) in a circumferential direction. It is defined that in a naturally expanded state of the lumen stent (100), a region which can be spanned by the restraining unit (31) of the lumen stent in the circumferential direction is the first region (100a), and a region which cannot be spanned by the restraining unit of the lumen stent in the circumferential direction is the second region (100b). The restraining point (33) is arranged in the second region (100b), and the imaging assembly (40) is configured to indicate a circumferential position of the restraining point (33). By providing the imaging assembly (40) to indicate the circumferential position of the restraining point (33), the lumen stent (100) can be prevented from placing the second region, which is traversed by the limiting rod (73), on a lesser curvature side of a blood vessel in a case that a semi-deployed state is to be deactivated, thereby avoiding endoleak at a distal end.

## Description

### Technical Field

The embodiments relate to the technical field of medical instruments, and in particular to, a lumen stent and a stent system.

### Background

Aortic aneurysm and aortic dissection are currently serious diseases that pose significant threats to human life safety. Without active treatment, the aortic aneurysm and the aortic dissection will continue to become large and eventually rupture, leading to severe complications and death. With the increasing number of patients with hypertension, hyperlipidemia, and hyperglycemia, the incidence rate of the aortic aneurysm and the aortic dissection also increases significantly. Traditional open surgical treatment for the aortic aneurysm and the aortic dissection involves a significant trauma, a high mortality rate, prolonged operation time, a high incidence rate of postoperative complications, and a high surgical difficulty. In contrast, thanks to characteristics of minimal trauma, fewer postoperative complications, shorter operation time, low surgical difficulty, and the like, an endovascular therapy has gradually become a primary manner for treating the aortic aneurysm and the aortic dissection. By implanting a covered stent in an aorta, a vascular lesion is isolated outside the stent, and a blood flow is confined to pass through an interior of the stent, thereby achieving a goal of vascular protection.

During the implantation of the covered stent, it is common that the stent has been partially apposed to a vascular wall but not completely deployed. This can easily lead to the "wind bag effect." The wind bag effect means that a proximal end of the stent has been apposed to the vascular wall while a distal end remains undeployed, which blocks a blood flow path. As the heart continues to pump blood over time, an increasing amount of blood is blocked at the position of the stent. As a result, the stent appears like a distended bag. The formation of the "wind bag effect" is extremely dangerous in a surgery because it blocks the blood flow. The heart continues to pump blood, leading to a persistent increase in a blood pressure. This can cause blood vessel rupture and an excessive cardiac load, and severely cause death of patients. After the "wind bag effect" is formed, the stent easily shifts, and cannot be deployed at a lesion site, or there is no sufficient anchoring region, so that the surgery fails.

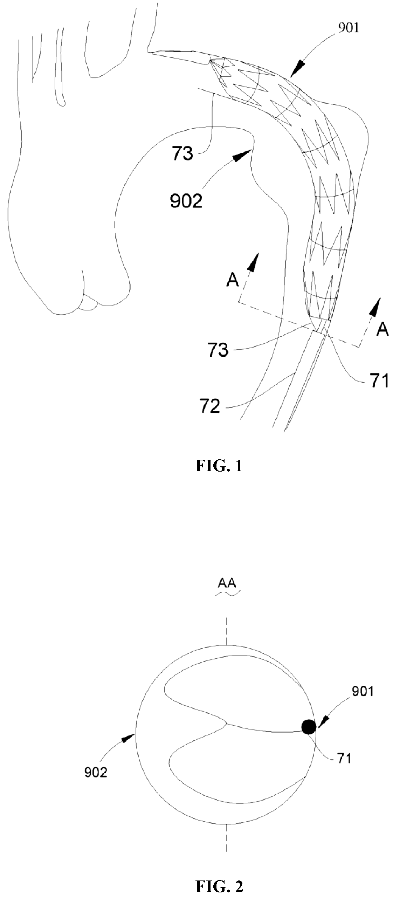

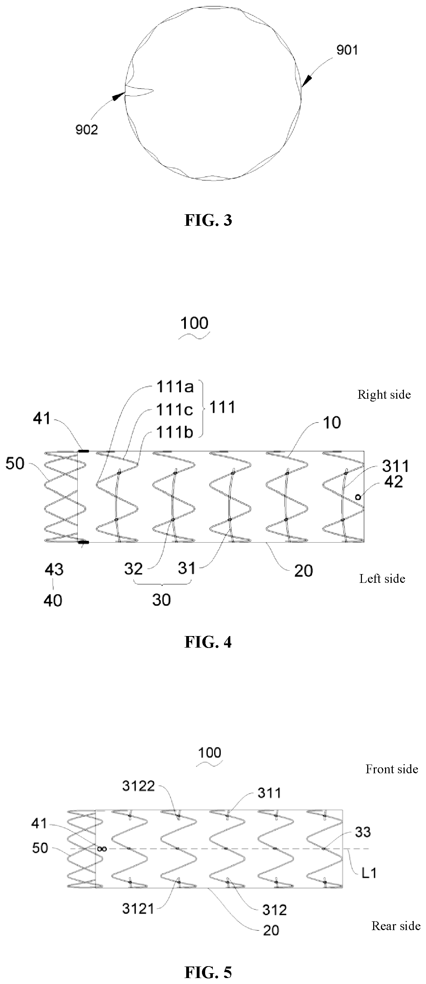

To solve the above problems, a semi-deployed structure can be arranged on an aortic covered stent to gradually deploy the stent, to avoid the formation of the "wind bag effect". In addition, when the stent is not closely apposed to the vascular wall, the axial position of the stent can be finely adjusted to enhance the accuracy of deploying the stent to the lesion site. However, during delivery of a thoracic aorta stent, since the path of a delivery guide wire is clung to a greater curvature side 901 of the blood vessel, if a limiting rod 73 is located on a lesser curvature side 902 of the blood vessel, as shown in FIG. 1, when the limiting rod 73 is withdrawn to deactivate a semi-restrained state to completely deploy the stent, in one aspect, the withdrawal of the limiting rod easily causes the proximal end of a lesser curvature side of the stent to shift toward the distal end. Consequently, after the covered stent is completely deployed, the proximal end of the graft is not closely apposed to the vessel wall, and endoleak at the proximal end occurs. In another aspect, particularly, a distal end of the lesser curvature side of the stent can be pressed by the limiting rod towards the greater curvature side before the withdrawal of the limiting rod, as shown in FIG. 1 to FIG. 2. This affects the form of the stent at the distal end before the stent is deployed. After the stent is deployed, incomplete spreading of the distal end of the stent easily occurs (a portion pressed by the limiting rod at a local position), and non-uniform folds are easily formed at this position, so that a large gap is formed between the non-uniform folds and the blood vessel, as shown in FIG. 3. In a severe case, endoleak at the distal end of the stent easily occurs.

### Summary

A technical problem to be solved in the present invention is how to provide a lumen stent to prevent endoleak because a semi-restrained structure possibly leads to insufficient close apposition of a distal end of the lumen stent.

The present invention provide a lumen stent. The lumen stent includes a stent main body, a semi-deployed structure and an imaging assembly. The semi-deployed structure includes a restraining unit and a restraining point. The lumen stent includes a first region and a second region in a circumferential direction. It is defined that in a naturally expanded state for the lumen stent, a region which can be spanned by the restraining unit of the lumen stent in the circumferential direction is the first region, and a region which cannot be spanned by the restraining unit of the lumen stent in the circumferential direction is the second region. The restraining point is arranged in the second region. The imaging assembly is configured to indicate a circumferential position of the restraining point.

In one embodiment, a first sub-region that is centrally symmetrical to the second region is provided in the first region; the imaging assembly includes a first imaging member;
the first imaging member is arranged in the second region; the central angle α between the axial line on which the first imaging member is located and the axial line in which the restraining point is located satisfies: 0° ≤ α ≤ 45°;
alternatively, the first imaging member is arranged in the first sub-region; the central angle α1 between the axial line on which the first imaging member is located and the axial line on which the restraining point is located satisfies: 135° ≤ α1 ≤ 180°;
alternatively, the lumen stent includes a second imaging member; the central angle α2 between the axial line on which the second imaging member is located and the axial line on which the restraining point is located satisfies: 45° ≤ α2 ≤ 135°; and the second imaging member is a non-axisymmetric structure.

In one embodiment, the range of the ratio of the central angle corresponding to the first region in the circumferential direction to the central angle corresponding to the second region in the circumferential direction is 1:1 to 4:1; it is defined that the central angle corresponding to the second region in the circumferential direction is β, and β satisfies: 72° ≤ β ≤ 180°; when β satisfies: 90° < β ≤ 180°, the restraining point is disposed within a region range of ± 45° of the angle bisector line of the second region; and when β satisfies: 72° ≤ β ≤ 90°, the restraining point is disposed in the second region.

In one embodiment, the restraining point includes at least one limiting member; when the lumen stent is radially compressed into a semi-restrained state, the restraining unit is able to pass through the limiting member;
the at least one limiting member and the first imaging member are on the same axial line;
alternatively, the at least one limiting member is closer to a middle position of the second region relative to the first imaging member in the circumferential direction.

In one embodiment, a plurality of limiting members are provided, and include a first limiting member and a second limiting member; and the deflection of the first limiting member relative to the second limiting member in the circumferential direction is less than or equal to 10°.

In one embodiment, the lumen stent further includes a covering film that covers the stent main body; the imaging assembly includes a first imaging member; the first imaging member is arranged in the second region; the central angle between the axial line on which the first imaging member is located and the axial line on which the restraining point is located is less than or equal to 45°; the lumen stent includes a second imaging member; the second imaging member is arranged on the axial line obtained by rotating 90° circumferentially from the first imaging member; and the first imaging member is arranged at a proximal end of the stent main body or the covering film, and/or the second imaging member is arranged at the distal end of the stent main body or the covering film.

In one embodiment, the first region further includes a first sub-region that is centrally symmetrical to the second region; the lumen stent further includes a third imaging member; and the circumferential position of the third imaging member is centrally symmetrical to the position of the first imaging member in the circumferential direction.

In one embodiment, the first imaging member and the third imaging member have different shapes, and the second imaging member is an axisymmetric structure or a non-axisymmetric structure;
alternatively, the first imaging member and the third imaging member have the same shape, and the second imaging member is a non-axisymmetric structure.

The present invention further provides a stent system, including a delivery device and the above lumen stent. The delivery device includes a sheath core assembly and a limiting rod; the restraining unit includes a diameter reduction line and a locking clasp assembly; and the limiting rod is able to extend along the restraining point in an axial direction of the lumen stent, so as to movably pass through the locking clasp assembly, to compress the lumen stent into a circumference formed by the diameter reduction line such that the lumen stent is restrained into a semi-restrained state.

In one embodiment, the semi-deployed structure further includes a limiting ring buckle; the limiting ring buckle is arranged on the stent main body in the circumferential direction; and the restraining unit passes through the limiting ring buckle in the circumferential direction such that the limiting ring buckle limits an axial position of the restraining unit.

A technical effect of one embodiment of the present invention is as follows: By providing the imaging assembly to indicate the circumferential position of the restraining point, the lumen stent can be prevented from placing the second region, which is traversed by the limiting rod, on a lesser curvature side of a blood vessel in the case that a semi-deployed state is to be deactivated, thereby avoiding endoleak at the distal end.

### Brief Description of the Drawings

FIG. 1 is a schematic diagram showing that, in the prior art, a failure of identification of a circumferential position of a restraining point of a lumen stent may result in the restraining point being deployed on a lesser curvature side;
FIG. 2 is a cross-sectional view of FIG. 1 in the direction A-A;
FIG. 3 is a schematic diagram of an apposed situation of a distal end of the lumen stent in the direction A-A after the lumen stent in FIG. 1 is completely deployed;
FIG. 4 is a schematic structural diagram (a naturally expanded state) of a lumen stent according to the present invention;
FIG. 5 is a schematic structural diagram of the lumen stent in FIG. 4 after it is rotated 90° in a circumferential direction (it is rotated 90° anticlockwise in a view from right to left shown in FIG. 4);
FIG. 6 is a schematic diagram (a naturally expanded state) of a restraining unit spanning a circumferential region of the lumen stent in FIG. 5 in a view from left to right;
FIG. 7 is a schematic diagram of partitioning of a first region and a second region in the view of FIG. 6;
FIG. 8 is a schematic diagram of a ± 45° region M of an angle bisector line of a second region in FIG. 7;
FIG. 9 is a schematic diagram of partitioning of a second region and a first sub-region in the view of FIG. 6;
FIG. 10 is a schematic structural diagram of a lumen stent (in a naturally expanded state) in another view according to the present invention;
FIG. 11 is a partial schematic structural diagram of FIG. 10 (showing a limiting member);
FIG. 12 is a schematic structural diagram of a lumen stent in a semi-restrained state according to the present invention;
FIG. 13 is a partial schematic structural diagram of FIG. 12 (showing cooperation between a restraining unit and a limiting rod);
FIG. 14 shows a stent system delivered to a predetermined position according to the present invention;
FIG. 15 shows a lumen stent being in a semi-restrained state after a sheath of a stent system is withdrawn according to the present invention;
FIG. 16 is an enlarged view of part E in FIG. 15; and
FIG. 17 is a schematic diagram of a lumen stent being completely deployed at a predetermined position according to the present invention.

100: lumen stent; 10: stent main body; 111: waveform ring; 111a: wave crest; 111b: wave trough; 111c: wave rod;
20: covering film;
30: semi-deployed structure; 31: restraining unit; 311: diameter reduction line; 312: locking clasp assembly; 3121: first locking clasp; 3122: second locking clasp; 32: limiting ring buckle; 33: restraining point; 34: middle fixing buckle;
40: imaging member; 41: first imaging member; 42: second imaging member; 43: third imaging member;
100a: first region; 100b: second region; 100c: first sub-region;
50: bare waveform circle;
70: delivery device; 71: sheath core assembly; 72: sheath; 73: limiting rod;
L1: axial line on which the first imaging member is located; and L2: axial line on which the restraining point is located.

### Detailed Description of the Invention

In order to facilitate understanding the present invention, the present invention will be described more comprehensively below with reference to related accompanying drawings. Preferred embodiments of the present invention are provided in the accompanying drawings. However, the present invention can be implemented in many different forms, and are not limited to the embodiments described herein. On the contrary, these embodiments are provided to make the content disclosed in the present invention understood more thoroughly and comprehensively.

It should be noted that when an element is referred to as being "fixed to" another element, the element can be directly on another component or there can be a centered element. When an element is considered to be "connected" to another element, the element can be directly connected to another element or there may be a centered element. The terms "inner", "outer", "left", "right", and similar expressions used herein are for illustrative purposes only and do not necessarily represent the only embodiment.

Unless otherwise defined, all technical and scientific terms used herein have the same meaning as those commonly understood by a person skilled in the art to which the present invention belongs. The terms used in this specification of the present invention are merely intended to describe objectives of the specific embodiments, but are not intended to limit the present invention. The term "and/or" used herein includes any and all combinations of one or more related listed items.

"Axial direction" means a length direction of a medical instrument during delivery. "Radial direction" generally means a direction which is perpendicular to the "axial direction" of the medical instrument. Based on this principle, an "axial direction" and a "radial direction" of any component of the medical instrument are defined. In addition, in the field of interventional medicine, it is usually defined that an end of a lumen stent that is close to the heart after deployment is a proximal end, and an end that is far from the heart is a distal end.

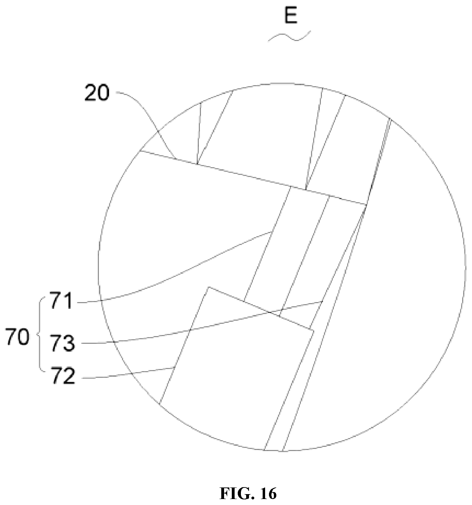

The present invention provides a lumen stent 100, as shown in FIG. 4 to FIG. 17. The lumen stent 100 includes a stent main body 10, a covering film 20, a semi-deployed structure 30, a restraining point 33, an imaging member 40, and a bare waveform circle 50. The stent main body 10 includes a plurality of waveform rings 111 spaced apart in an axial direction. Each waveform ring 111 includes a plurality of wave crests 111a and wave troughs 111b, and a plurality of connection rods respectively connected to adjacent wave crests 111a and wave troughs 111b. The covering film 20 covers the plurality of waveform rings 111 of the stent main body 10 to form a hollow endoluminal structure. The distal end of the bare waveform circle 50 is connected to the proximal end of the covering film 20, as shown in FIG. 4 to FIG. 5. In other embodiments, the bare waveform circle 50 may not be provided, and the lumen stent can be directly semi-restrained and loaded to a loading position formed between a sheath core assembly 71 and a sheath 72, without limitation here. It can be understood that the plurality of waveform rings 111 may have the same or similar waveform shapes, or may be waveform rings with different waveforms. The waveforms of the waveform rings 111 can be set as needed, and the number of waveforms, the wave height, and the like in each waveform ring 111 can be set as needed.

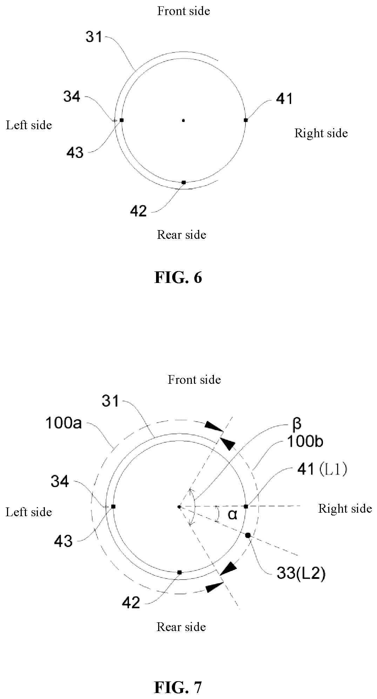

As shown in FIG. 4 to FIG. 5, the semi-deployed structure 30 includes restraining units 31, limiting ring buckles 32, and limiting members 330. The restraining units 31 pass through the limiting ring buckles 32 in a circumferential direction. The restraining units 31 include diameter reduction lines 311 and locking clasp assemblies 312. The locking clasp assemblies 312 are connected to the diameter reduction lines 311 and are formed at two ends of the diameter reduction lines 311. In this embodiment, each diameter reduction line 311 is a double-stranded wire. Each locking clasp assembly 312 is a folded end of the diameter reduction line 311. In other embodiments, the diameter reduction line 311 may be two sections of double-stranded wires extending from a middle axial line of a first region 100a toward two circumferential ends of the first region 100a. A middle fixing buckle 34 is arranged on the middle axial line of the first region 100a, and the two sections of double-stranded wires respectively circumferentially extend from the middle fixing buckle 34 toward a second region 100b to an edge of the first region 100a, as shown in FIG. 6 to FIG. 9. Providing the middle fixing buckle 34 on the middle axial line of the first region 100a can make the restraining of the diameter reduction line 311 more stable.

As shown in FIG. 4 to FIG. 5, the limiting ring buckles 32 are arranged on the bracket body 10 in the circumferential direction and may be fixed on the waveform rings 111 or the covering film 20. In this embodiment, a plurality of limiting ring buckles 32 are circumferentially spaced apart and fixed in middle portions of part of wave rods 111c of the waveform rings 111 (a wave crest 111a is formed at an end point of the proximal end between two adjacent wave rods 111c, and each limiting ring buckle 32 is arranged on at least one of two adjacent wave rods 111c). Each diameter reduction line 311 sequentially passes through the plurality of limiting ring buckles 32 on the corresponding waveform rings in the circumferential direction, so that the limiting ring buckles 32 define the diameter reduction lines 311 in a middle region in an axial direction of the waveform rings 111 to prevent a non-uniform radial constraining force of the waveform rings 111, thereby facilitating circumferential constraining performed by the restraining unit 31 on the stent main body 10. The locking clasp assembly 312 includes a first locking clasp 3121 and a second locking clasp 3122 that are connected to two ends of the diameter reduction line. The first locking clasp 3121 and the second locking clasp 3122 are both annular end portions formed by folding the diameter reduction line 311. After the lumen stent 100 is radially compressed, the first locking clasp 3121 passes through each limiting member 330 in the second region 100b, and a limiting rod 73 of a delivery device 70 axially passes through the first locking clasp 3121 to limit the first locking clasp 3121 at the limiting member 330. The limiting rod 73 also needs to pass through the second locking clasp 3122 to radially compress the lumen stent 100 into the sheath 72 after the lumen stent 100 is subjected to diameter reduction to a semi-deployed state.

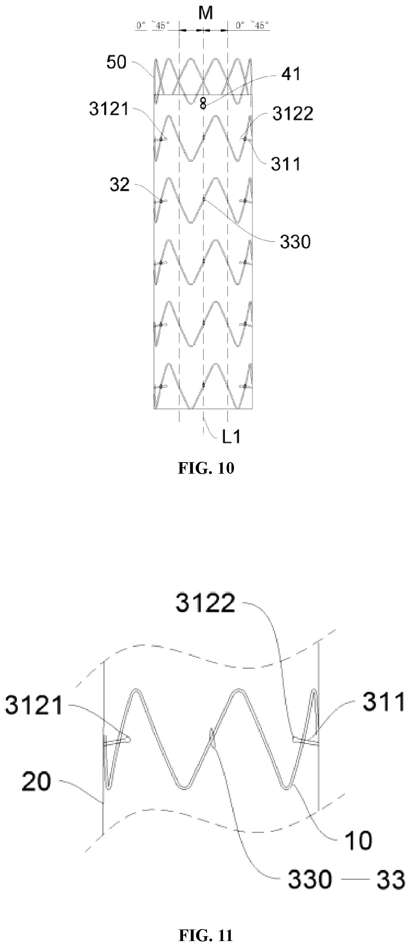

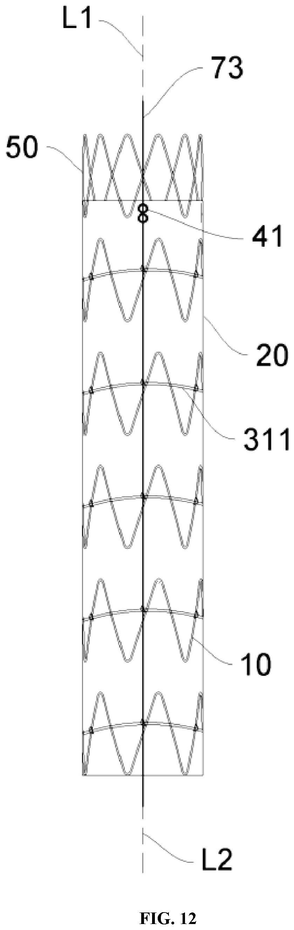

As shown in FIG. 6 to FIG. 9 combined with FIG. 10 to FIG. 13, the semi-deployed structure 30 can be configured to constrain the circumferential direction of the stent main body 10. In this embodiment, the semi-deployed structure 30 is arranged on an outer surface of the lumen stent 100, as shown in FIG. 6. When the sheath 72 is withdrawn such that the lumen stent 100 compressed in the delivery device 70 is deployed from the sheath 72, due to the radial constraining of the semi-deployed structure, the lumen stent 100 is in the semi-deployed state, as shown in FIG. 12 to FIG. 13. In this case, an outer diameter of the lumen stent 100 is less than an inner diameter of a blood vessel due to the radial constraining, and thus is not closely apposed to an inner wall of a blood vessel. Therefore, in the semi-deployed state of the lumen stent 100, the circumferential position of the restraining point 33 can still be determined based on a position of an imaging assembly, so as to finely adjust axial and circumferential positions of the lumen stent 100. After both the axial and circumferential positions are adjusted to expected positions, radial constraining of a semi-restrained structure is deactivated, and the lumen stent 100 is changed from the semi-deployed state to a completely deployed state and is closely apposed to the inner wall of the blood vessel.

FIG. 4 to FIG. 9 show a front side, a rear side, a left side, and a right side of the stent being opposite orientations to front, back, left, and right of a patient. The illustration is merely for the purpose of facilitating the description of the orientation of the stent. A side visible in FIG. 4 (i.e. facing a reader on the paper) is the rear side, corresponding to a bottom (rear side) of FIG. 5. A side not visible in FIG. 4 (i.e. the back of the paper) is the front side, corresponding to the top (front side) of FIG. 5. Similarly, a side visible in FIG. 5 (i.e. facing a reader on the paper) is the right side, corresponding to a top (right side) of FIG. 4. A side not visible in FIG. 5 (i.e. the back of the paper) is the left side, corresponding to the bottom (left side) of FIG. 4.

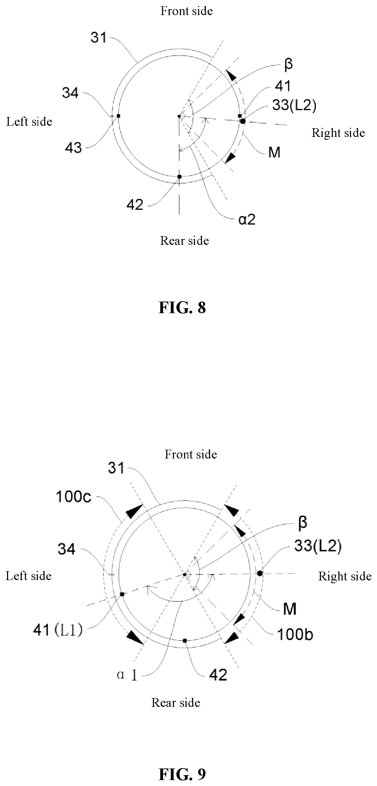

As shown in FIG. 6 to FIG. 9, the lumen stent 100 includes a first region 100a and a second region 100b in a circumferential direction. It is defined that when the lumen stent 100 is in a naturally expanded state, a region which can be spanned by the restraining unit 31 of the lumen stent 100 in the circumferential direction is the first region 100a, and a region which cannot be spanned by the restraining unit 31 of the lumen stent 100 in the circumferential direction is the second region 100b. The imaging assembly 40 includes a first imaging member 41. The first imaging member 41 is arranged in the second region 100b. The central angle α between an axial line L1 on which the first imagingt member 41 is located and an axial line L2 in which the restraining point 33 is located satisfies: 0° ≤ α ≤ 45°, as shown in FIG. 7. In other embodiments, as shown in FIG. 9, a first sub-region 100c that is centrally symmetrical to the second region 100b is provided in the first region 100a. A central angle α1 between the axial line L1 on which the first imaging member 41 is located and the axial line L2 on which the restraining point 33 is located satisfies: 135° ≤ α1 ≤ 180°. The maximum value of α1 is 180°. Therefore, FIG. 9 only shows that the first imaging member is located at a lower position in the first sub-region 100c in the figure, and the angle α1 formed is within an lower half circle; In other embodiments, the angle α1 formed is within an upper half circle (omitted in the figure). The first imaging member 41 can be arranged in the second region 100b or the first sub-region 100c to distinguish a left side of the lumen stent 100 from a right side. Meanwhile, the restraining point 33 is arranged on the same side as or an opposite side to the first imaging member 41, so that the first imaging member 41 can be configured to indicate the circumferential position of the restraining point 33.

Alternatively, the lumen stent 100 may include a second imaging member 42. A central angle α2 between an axial line on which the second imaging member 42 is located and the axial line L2 on which the restraining point 33 is located satisfies: 45° ≤ α2 ≤ 135°. As shown in FIG. 8, the second imaging member 42 is a non-axisymmetric structure, to distinguish front and rear orientations of the lumen stent 100, so that the restraining point 33 is arranged on a specific side (such as the right side in FIG. 8) of the second imaging member 42. Thus, the imaging imaging member 42 can be configured to indicate the circumferential position of the restraining point 33. Similarly, the lumen stent 100 can be prevented from placing the second region 100b, which is traversed by the limiting rod 73, on a lesser curvature side of a blood vessel in the case that the semi-deployed state is to be deactivated, thereby avoiding endoleak at the distal end. In addition, the problem of insufficient close apposition caused by shifting of the proximal end of a lesser curvature side of the stent due to the withdrawal of the limiting rod can also be prevented, thereby avoiding endoleak at the proximal end.

The first imaging member 41 is arranged at the proximal end of the stent main body 10 or the covering film 20, and the imaging assembly is arranged at the proximal end of the stent main body 10 or the covering film 20. This can simultaneously indicate a position of an end portion of the proximal end of the stent. The second imaging member 42 is arranged at the proximal or distal end of the lumen stent 100. The second imaging member 42 can be arranged on the stent main body 10 or the covering film 20. Only the first imaging member 41 can be provided to distinguish the first side of the lumen stent 100 from the second side. Alternatively, only the second imaging member 42 can be provided to distinguish between the front part of the lumen stent 100 from the rear part. When only the second imaging member 42 is provided, the second imaging member 42 needs to be a non-axisymmetric structure to distinguish the front part of the lumen stent 100 from the rear part. Alternatively, the first imaging member 41 and the second imaging member 42 can be provided simultaneously. In other embodiments, the lumen stent 100 may further include a third imaging member 43. The first imaging member 41 is arranged in the second region 100b. The third imaging member 43 is centrally symmetrical to the first imaging member 41 in the circumferential direction, and/or the second imaging member 42 is rotated 90° from the first imaging member 41 in the circumferential direction. The second imaging member 42 may be arranged at a proximal or distal end of the covering film 20 in a sutured manner.

When the first imaging member 41 and the third imaging member 43 are different in shape, it is possible to distinguish the left part of the lumen stent 100 from the right part. The second imaging member 42 can be an axisymmetric structure or a non-axisymmetric structure. The first imaging member 41 and the third imaging member 43 have the same shape, and the second imaging member 42 needs to be configured as a non-axisymmetric structure to distinguish the front part of the lumen stent 100 from the rear part. Alternatively, when the lumen stent 100 is implanted into a blood vessel, the second region 100b is closely apposed to a greater curvature side of the blood vessel to deactivate the restraining of the semi-deployed structure 30 of the lumen stent 100, so that the lumen stent 100 is completely deployed. In one embodiment, the non-axisymmetric structure of the second imaging member 42 can be a non-axisymmetric recognizable pattern including a closed structure, such as a "6" shape, a "G" shape, and a "9" shape.

As shown in FIG. 4 to FIG. 5 combined with FIG. 10 to FIG. 13, in this embodiment, the lumen stent 100 includes a first imaging member 41, a second imaging member 42, and a third imaging member 43. The first imaging member 41 and the third imaging member 43 are arranged at the proximal end of the covering film 20, and the second imaging member 42 is arranged at the distal end of the covering film 20. Each imaging member can be fixed to the covering film 20 in a sutured manner. The first imaging member 41 and the second imaging member 42 have different shapes. The first imaging member 41 is arranged in the second region 100b. The first imaging member 41 can be configured to be in an "8" shape. A plurality of limiting members 330 form an axial constraining point 33 in the axial direction and are sequentially arranged on a middle axial line of the second region 100b, thereby forming the axial line L2 on which the restraining point 33 is located. In this embodiment, the first imaging member is also arranged on the middle axial line of the second region 100b, so the middle axial line of the second region 100b is the axial line L1 on which the first imaging member 41 is located. That is, the axial line L1 on which the first imaging member 41 is located and the axial line L2 on which the restraining point 33 is located are the same axial line. In this embodiment, α=0°., as shown in FIG. 10. The first imaging member 41 and the limiting members 330 are arranged on the same axial line (or the first imaging member 41 is deflected not more than 10° relative to the axial line on which the limiting members 330 are located), and the first imaging member 41 is sutured to the proximal end of the covering film 20. The limiting members 330 are configured as suture buckles which are sutured to the covering film 20 around middle positions of the wave rods 111c of waveform rings 111 corresponding to the limiting members 330 or are directly sutured to the covering film 20 next to the middle positions of the wave rods 111c without surrounding the wave rods 111c, to leave gaps for the restraining units 31 to pass through. The third imaging member 43 may be configured to be in a "0" shape and is centrally symmetrical to the first imaging member 41 in the circumferential direction. Different structures of the first imaging member 41 and the second imaging member 42 can distinguish left and right orientations (the first side and the second side) of the lumen stent 100. The second imaging member 42 may be configured to be in a "0" shape, and is arranged the distal end of the covering film 20 of the lumen stent 100. Moreover, the second imaging member 42 is rotated 90° from the first imaging member 41 in the circumferential direction. In an imaging view shown in FIG. 4, the second imaging member 42 is located on a perpendicular bisector of a connecting line of the first imaging member 41 and the third imaging member 43, and can form a three-dimensional structure with the first imaging member 41 and the third imaging member 43. It is convenient to determine whether the distal end of the stent is uniformly spread in the circumferential direction after the complete deployment of the lumen stent 100. In other embodiments, only the first imaging member 41 can be provided, or only the second imaging member 42 with the non-axisymmetric structure can be provided, as long as the imaging assembly can indicate the circumferential position of the restraining point 33, so that the restraining point 33 of the lumen stent 100 in a semi-restrained state can be adjusted in the circumferential direction to be closely apposed to a greater curvature side of a blood vessel, without limitation.

Generally, two ends of each restraining unit 31 are releasable restrained to the restraining point 33, so that the semi-deployed structure 30 semi-restrain the stent main body 10 in the circumferential direction. When the limiting rod 73 is arranged in the delivery device 70 to limit the restraining units 31, the limiting rod 73 is generally axially arranged at the restraining point 33 in the second region 100b, so that two ends of the diameter reduction lines 311 are limited in the second region 100b to radially compress the lumen stent 100. In this embodiment, the locking clasp assemblies 312 at the two ends of the diameter reduction lines 311 are close to each other at a middle position of the second region 100b. After the limiting rod 73 passes through the locking clasp assemblies 312 at the two ends, the waveform rings 111 of the lumen stent 100 are compressed. The limiting rod 73 sequentially passes through the locking clasp assemblies 312 at the two ends of the diameter reduction lines 311 corresponding to the waveform rings, thereby compressing the entire lumen stent 100 into the semi-restrained state along its radius. The lumen stent 100 further includes a first side and a second side in the circumferential direction. The first side and the second side occupy 180° of the circumferential direction, and the second region 100b is located within a range of a ± 45° region M of an angle bisector line of the first side. The first side of the lumen stent is located on a greater curvature side of an aortic arch when implanted into the blood vessel, and the second side is located on a lesser curvature side of the aortic arch when implanted into the blood vessel. The arrangement of the imaging assembly can be configured to indicate the circumferential position of the restraining point 33, so that the first side and the second side of the lumen stent 100 can be distinguished during implantation of the lumen stent 100. During the adjustment of the circumferential direction of the lumen stent 100 in the semi-deployed state, the restraining point 33 of the second region 100b can correspond to the greater curvature side of the blood vessel, so that during the deactivation of the semi-deployed state of the lumen stent 100, the second region 100b of the lumen stent 100 is closely apposed to the greater curvature side of the blood vessel, causing the limiting rod 73 to be withdrawn while it is apposed to the greater curvature side, thus preventing endoleak at the proximal end because of insufficient close apposition of the proximal end after the covered stent is completely deployed due to shifting of the proximal end of the second side of the stent toward the distal end during the withdrawal of the limiting rod 73 to deactivate the semi-deployed state. In another aspect, the distal end of the second side of the lumen stent 100 can be prevented from being pressed by the limiting rod 73 toward the greater curvature side before the withdrawal of the limiting rod 73 is completed, to ensure that the distal end of the lumen stent 100 has a good form in the semi-deployed state and avoid incomplete spreading of the distal end of the second side of the lumen stent 100 after the stent is deployed, thereby avoiding formation of non-uniform folds and avoiding endoleak at the distal end of the stent.

It can be understood that if a diameter of a circumcircle of a cross section of the lumen stent 100 is too large when the lumen stent 100 is in the semi-deployed state, the lumen stent 100 is easily apposed to the inner wall of the blood vessel in the semi-deployed state. This is not conducive to axial and circumferential adjustment on the lumen stent 100. If the diameter of the circumcircle of the cross section of the lumen stent 100 is too small when the lumen stent 100 is in the semi-deployed state, even if the circumferential or axial position is adjusted, there may still be a significant circumferential and axial positioning deviation due to a large radial change in the stent after the semi-deployed state is deactivated. As a result, the effect of the semi-deployed structure 30 is poor. In this embodiment, the ratio of the diameter of the circumcircle of the cross section when the lumen stent 100 is in the semi-deployed state to the diameter of the circumcircle of the cross section when the lumen stent 100 naturally expands is 0.5 to 0.8.

Since the perimeter of the circumcircle of the cross section in the semi-deployed state is the circumferential length of each diameter reduction line 311, the ratio of the diameter of the circumcircle of the cross section when the lumen stent 100 is in the semi-deployed state to the diameter of the circumcircle of the cross section when the lumen stent 100 naturally expands is equal to the ratio of the central angle corresponding to the first region 100a in the circumferential direction to the circumference angle of 360 degrees. When the ratio of the diameter of the circumcircle of the cross section when the lumen stent is in the semi-deployed state to the diameter of the circumcircle of the cross section when the lumen stent naturally expands is 0.5, the central angle corresponding to the first region in the circumferential direction is 180°. When the ratio of the diameter of the circumcircle of the cross section when the lumen stent is in the semi-deployed state to the diameter of the circumcircle of the cross section when the lumen stent naturally expands is 0.8, the central angle corresponding to the first region in the circumferential direction is 288°, and the central angle corresponding to the second region in the circumferential direction is 72°. It is defined that the central angle covered by the second region 100b is β, as shown in FIG. 7. The central angle corresponding to the first region 100a in the circumferential direction is 360° - β, where β satisfies: 72° ≤ β ≤ 180°. When β satisfies: 90° < β ≤ 180°, the first imaging member 41 is arranged within the range of the ± 45° region M of the angle bisector line of the second region 100b or the first sub-region 100c. When βsatisfies: 72° ≤ β ≤ 90°, the first imaging member 41 is arranged in the second region 100b or the first sub-region 100c to distinguish the first side of the lumen stent 100 from the second side, and the limiting rod 73 in the second region 100b is closer to a middle position of the first side, so that when the lumen stent 100 is in the semi-deployed state, under the indication of the imaging assembly 40, the limiting rod 73 located at the restraining point 33 is better closely apposed to the greater curvature side of the blood vessel.

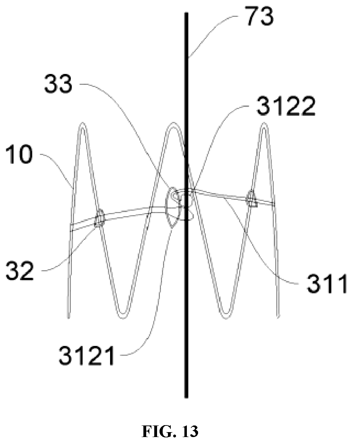

The restraining point 33 of the lumen stent includes at least one limiting member 330. The limiting member 330 can be configured to limit the limiting rod 73 at the restraining point. In this embodiment, the restraining point 33 of the lumen stent 100 includes a plurality of limiting members. The limiting members are axially arranged in the second region 100b. The limiting members are generally arranged within the range of the ± 45° region M of the angle bisector line of the second region 100b. Distances from the locking clasp assemblies 312 at the two ends of the diameter reduction lines 311 in the circumferential direction are equal or approximately equal, so that in the semi-deployed state of the lumen stent 100, the limiting rod 73 in the second region 100b is closer to a middle position of the greater curvature side of the blood vessel. When the lumen stent 100 is radially compressed into the semi-restrained state, one end of each restraining unit 31 can pass through each limiting member, and then the limiting rod 73 can sequentially pass through the locking clasp assemblies 312 at the two ends of the restraining unit 31. The quantity of limiting members can be specifically determined based on the quantity of waveform rings 111 of the stent and a degree of extension desired in semi-restraining in the axial direction. The plurality of limiting members are spaced apart in the axial direction to form the restraining point 33, and the central angle between the axial line on which the restraining point 33 is located and the axial line L1 on which the first imaging member 41 is located is α. In addition, the limiting members are closer to the middle position of the second region 100b relative to the first imaging member 41 in the circumferential direction. When the lumen stent 100 is radially compressed into the semi-deployed state, the locking clasp assembly 312 at one end of each diameter reduction line 311 passes through a limiting member corresponding to the locking clasp assembly 312 in the circumferential direction. Then, the limiting rod 73 passes through the locking clasp assemblies 312 such that the limiting members limit a circumferential position of the limiting rod 73, thus limiting the limiting rod 73 at the limiting members, as shown in FIG. 13. When α=0°, the limiting members and the first imaging member 41 are on the same axial line L1, as shown in FIG. 10 to FIG. 11 combined with FIG. 12 to FIG. 13, and the plurality of limiting members are arranged in the axial direction within 10° of left and right sides of the middle axial line L1 of the second region 100b. In one embodiment, the limiting members and the first imaging member 41 are both located on the middle axial line of the second region 100b, so that distances between the limiting members and the locking clasp assemblies 312 at the two ends of the diameter reduction lines 311 in the circumferential direction are approximately equal. When the lumen stent 100 is in the semi-deployed state, a compressed state of the lumen stent 100 is uniform, and the semi-restrained state is more stable.

The plurality of limiting members can be sequentially arranged along the same axial line on the waveform rings 111 or on the covering film 20 corresponding to the middle positions of the wave rods 111c of the waveform rings 111, or can be deflected slightly, as long as the limiting members do not affect the uniform compression and deployment in the semi-restrained state. No limitation is made here. The limiting members include a first limiting member and a second limiting member, as long as the central angle between the axial line on which any limiting member (restraining point 33) is located and the axial line L1 on which the first imaging member 41 is located satisfies α, and the circumferential deflection of the first limiting member relative to the second limiting member is less than or equal to 10°, so that the limiting rod 73 is approximately in the axial direction.

The present invention further provides a stent system, including a delivery device 70 and the above lumen stent 100. The delivery device 70 includes a sheath core assembly 71, a sheath 72, and a limiting rod 73. The limiting rod 73 extends in the axial direction of the second region 100b and is movably connected to the locking clasp assemblies 312. The lumen stent 100 is radially compressed into a loading region formed between the sheath 72 and the sheath core assembly 71.

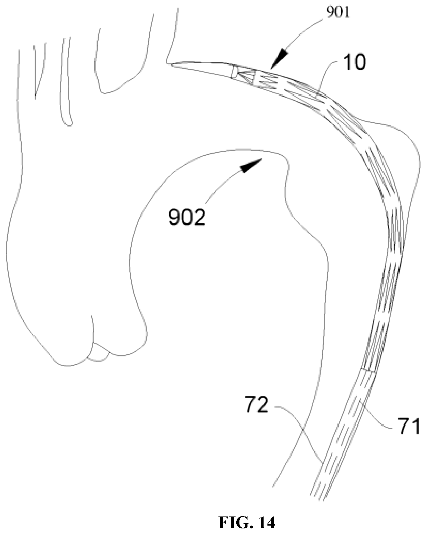

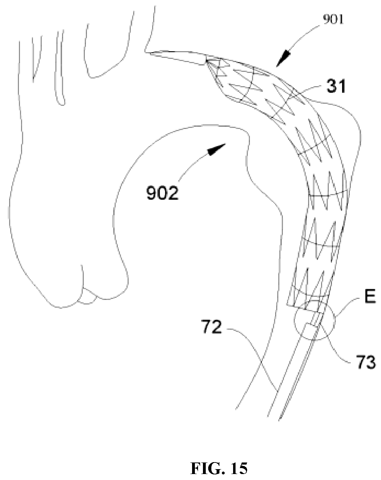

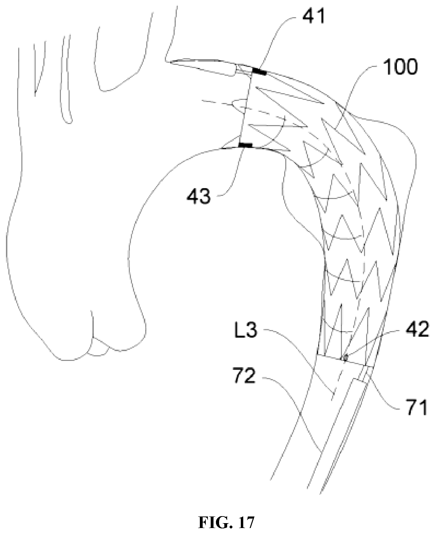

Implantation of a thoracic aorta stent system is used as an example. As shown in FIG. 14 to FIG. 17, when the stent system is delivered to a predetermined position, the sheath 72 is withdrawn, and the lumen stent 100 is not completely deployed to the semi-deployed state due to the effect of the semi-restrained structure, which can prevent a wind bag effect. As shown in FIG. 15, the axial position of the lumen stent 100 may be finely adjusted to make the deployment position of the lumen stent 100 more accurate. Meanwhile, based on the position of the first imaging member 41, the circumferential position of the lumen stent 100 is adjusted, so that the side on which the limiting rod 73 of the stent system is located is closely apposed to the greater curvature side of a blood vessel, as shown in FIG. 15 to FIG. 16, to prevent endoleak because of insufficient close apposition of the proximal end of the lumen stent 100 due to shifting of the proximal end of the lesser curvature side of the stent toward a distal end during withdrawal of the limiting rod 73 to deactivate the semi-deployed state. In another aspect, it can also prevent the limiting rod 73 on the lesser curvature side from pressing the distal end of the lesser curvature side of the lumen stent 100 and prevent the distal end of the lesser curvature side of the lumen stent 100 from always being in a state of being recessed towards the greater curvature side before the stent is completely deployed, thereby preventing the problem of non-uniform spreading of the distal end of the lumen stent 100 after the stent is completely deployed and avoiding endoleak at the distal end of the stent. After the limiting rod 73 is withdrawn, the semi-restrained state is deactivated, and the lumen stent 100 is completely deployed, as shown in FIG. 17. In this embodiment, when the lumen stent 100 is completely deployed, a perpendicular bisector L3 (i.e., a centerline of the blood vessel) of the first imaging member 41 and the third imaging member 43 is defined, and whether the distal end of the lumen stent 100 is uniformly spread can be determined based on a position of the second imaging member 42 relative to the perpendicular bisector L3 of the first imaging member 41 and the third imaging member 43.

In other embodiments, the limiting rod 73 can also pass through the limiting members, so that the limiting members limit the limiting rod 73 to the axial line (i.e., the restraining point) on which the limiting members are located. The limiting rod 73 also passes through the first locking clasp 3121 and the second locking clasp 3122. Meanwhile, the first locking clasp 3121 and the second locking clasp 3122 are limited to the axial line on which the limiting members are located, which can achieve bundling of the semi-restrained structure. There is no limitation here. At least one of the locking clasp assembly 312 or the limiting rod 73 needs to pass through the limiting member to achieve an arrangement position of the limiting member, namely the restraining point 33.

The various technical features in the foregoing embodiments may be randomly combined. For concise description, not all possible combinations of the various technical features in the above embodiments are described. However, provided that combinations of these technical features do not conflict with each other, the combinations of the various technical features are considered as falling within the scope of this specification.

The foregoing embodiments merely express several embodiments of the present invention. The descriptions thereof are relatively specific and detailed, but are not understood as limitations on the scope of the present invention. It should be pointed out that a person of ordinary skill in the art can also make several transformations and improvements without departing from the idea of the present invention. These transformations and improvements fall within the protection scope of the present invention. Therefore, the protection scope of the patent of the present invention shall be subject to the appended claims.

## Claims

1. A lumen stent, comprising a stent main body, a semi-deployed structure, and an imaging assembly, wherein the semi-deployed structure comprises a restraining unit and a restraining point; the lumen stent comprises a first region and a second region in a circumferential direction; it is defined that in a naturally expanded state of the lumen stent, a region which can be spanned by the restraining unit of the lumen stent in the circumferential direction is the first region, and a region which cannot be spanned by the restraining unit of the lumen stent in the circumferential direction is the second region; the restraining point is arranged in the second region; and the imaging assembly is configured to indicate a circumferential position of the restraining point.

2. The lumen stent according to claim 1, wherein a first sub-region that is centrally symmetrical to the second region is provided in the first region; the imaging assembly comprises a first imaging member;
the first imaging member is arranged in the second region; a central angle α between an axial line on which the first imaging member is located and an axial line in which the restraining point is located satisfies: 0° ≤ α ≤ 45°;
alternatively, the first imaging member is arranged in the first sub-region; a central angle α1 between the axial line on which the first imaging member is located and the axial line on which the restraining point is located satisfies: 135° ≤ α1 ≤ 180°;
alternatively, the lumen stent comprises a second imaging member; a central angle α2 between an axial line on which the second imaging member is located and the axial line on which the restraining point is located satisfies: 45° ≤ α2 ≤ 135°; and the second imaging member is a non-axisymmetric structure.

3. The lumen stent according to claim 2, wherein a range of a ratio of a central angle corresponding to the first region in the circumferential direction to a central angle corresponding to the second region in the circumferential direction is 1:1 to 4:1; it is defined that the central angle corresponding to the second region in the circumferential direction is β, and β satisfies: 72° ≤ β ≤ 180°; when β satisfies: 90° < β ≤ 180°, the restraining point is disposed within a region range of ± 45° of an angle bisector line of the second region; and when β satisfies: 72° ≤ β ≤ 90°, the restraining point is disposed in the second region.

4. The lumen stent according to claim 2, wherein the restraining point comprises at least one limiting member; when the lumen stent is radially compressed into a semi-restrained state, the restraining unit is able to pass through the limiting member;
the at least one limiting member and the first imaging member are on the same axial line;
alternatively, the at least one limiting member is closer to a middle position of the second region relative to the first imaging member in the circumferential direction.

5. The lumen stent according to claim 4, wherein a plurality of limiting members are provided, and comprise a first limiting member and a second limiting member; and a deflection of the first limiting member relative to the second limiting member in the circumferential direction is less than or equal to 10°.

6. The lumen stent according to claim 1, wherein the lumen stent further comprises a covering film that covers the stent main body; the imaging assembly comprises a first imaging member; the first imaging member is arranged in the second region; a central angle between an axial line on which the first imaging member is located and an axial line on which the restraining point is located is less than or equal to 45°; the lumen stent comprises a second imaging member; the second imaging member is arranged on an axial line obtained by rotating 90° circumferentially from the first imaging member; and the first imaging member is arranged at a proximal end of the stent main body or the covering film, and/or the second imaging member is arranged at a distal end of the stent main body or the covering film.

7. The lumen stent according to claim 6, wherein the first region further comprises a first sub-region that is centrally symmetrical to the second region; the lumen stent further comprises a third imaging member; and a circumferential position of the third imaging member is centrally symmetrical to a position of the first imaging member in the circumferential direction.

8. The lumen stent according to claim 7, wherein the first imaging member and the third imaging member have different shapes, and the second imaging member is an axisymmetric structure or a non-axisymmetric structure;
alternatively, the first imaging member and the third imaging member have the same shape, and the second imaging member is a non-axisymmetric structure.

9. A stent system, comprising a delivery device and a lumen stent according to any one of claims 1 to 8, wherein the delivery device comprises a sheath core assembly and a limiting rod; the restraining unit comprises a diameter reduction line and a locking clasp assembly; and the limiting rod is able to extend along the restraining point in an axial direction of the lumen stent, so as to movably pass through the locking clasp assembly, to compress the lumen stent into a circumference formed by the diameter reduction line such that the lumen stent is restrained into a semi-restrained state.

10. The stent system according to claim 9, wherein the semi-deployed structure further comprises a limiting ring buckle; the limiting ring buckle is arranged on the stent main body in the circumferential direction; and the restraining unit passes through the limiting ring buckle in the circumferential direction such that the limiting ring buckle limits an axial position of the restraining unit.
